# EUROPEAN PATENT APPLICATION

(11) **EP 3 821 877 A1**
(43) Date of publication of application: **19.05.2021**
(21) Application number: 20207911.7
(22) Date of filing: 16.11.2020
(51) Int. Cl.: A61K 8/9789, A61K 8/46, A61K 8/34, A61K 8/44, A61Q 19/00

(54) **WATER-FREE COSMETIC COMPOSITION**

(30) Priority: 14.11.2019 IT 201900021192
(71) Applicant: Marie Danielle-Linea Hotel S.r.l., 20145 Milano (MI) (IT)
(72) Inventor: TRAMONTANA, Fabio, 20145 Milano (MI) (IT)
(74) Representative: Brunacci, Marco

(57) **Abstract**

The present invention relates to a water-free cosmetic composition comprising from 20% to 60% of fruit extract by weight of the total weight to promote the natural mechanisms of skin moisturizing as well as obtaining an antioxidant effect for the skin without using the synthetic fragrances.

## Description

### Technical Field

The present invention relates to a water-free cosmetic composition, its preparation process and its use.

### Background Art

So-called "water-free" cosmetic skin care compositions are widely known and mainly used for skin or lip care or e.g. as balms. In such cases, the absence of water in the formulation makes it possible to obtain moisture indirectly, e.g. by generating an insulating film, thanks to the use of film-forming substances, such as e.g. oils, butters, etc., which prevent the loss of moisture at the point where the acid mantle (MAI) is located, thus making the skin more hydrated and therefore more elastic.

The water-free cosmetic compositions with moisturizing/protective action can also provide for a direct rehydration by means of emulsions the oily part of which penetrates the skin and then reintegrates the necessary water.

The Applicant realized that known cosmetic compositions do, however, have several drawbacks. First of all, in order to obtain high moisturizing benefits, these compositions are mostly made with complex processes and formulations.

In addition, to obtain certain antioxidant properties, most of the time preservatives have to be added to determine proven chemical imbalances which are toxic to the skin and/or harmful to the environment. On the other hand, it is extremely difficult to eliminate these components which in fact allow for an adequate moisturizing of the skin.

### Description of the Invention

The Applicant therefore felt the need to create a new formulation of a cosmetic composition that avoids as much as possible the use of preservatives or synthetic products while keeping at the same time high moisturizing characteristics.

The Applicant has discovered that the use of a water-free cosmetic composition comprising specific components, in particular concentration ranges intended to work synergistically with high amounts of fruit extracts allows promoting the natural mechanisms of skin moisturizing as well as obtaining an antioxidant effect for the skin without the use of synthetic fragrances.

The present invention therefore relates, in its first aspect, to a water-free cosmetic composition according to claim 1.

According to a further aspect, the present invention relates to a process for the preparation of a cosmetic composition according to claim 7.

In a further aspect, the present invention relates to the use of a cosmetic composition to carry out a cosmetic epidermis moisturizing and skin preservation treatment according to claim 12.

Further characteristics and advantages of the cosmetic composition, of the process and of the use of the composition according to the present invention will result from the description below of a preferred embodiment.

In the experimental tests conducted by the Applicant, the formulation according to the present invention proved to be extremely effective in complying with the synergy of all components.

Other characteristics and advantages of the present invention will be more evident from the description of a preferred, but not exclusive embodiment specified below.

### Embodiments of the Invention

In the following of the present description and in the subsequent claims, the term "water-free" attributed to the formulation of the inventive cosmetic composition means that it does not contain water (whether added or not) or any other aqueous or polar solvent in any state, e.g. free or emulsified, comprising instead the various substances listed in the present description.

In the cosmetic composition to which the present invention relates, the content of fruit extract is comprised between 20% and 60%, preferably between 35% and 45% by weight of the total weight of the composition. Advantageously, the fruit extract is orange extract. In particular, the orange extract gives this composition effective skin defense properties, thus reducing oxidation phenomena, preventing skin aging and contributing to the softness of the skin coating layer left by the product on the skin after administration.

In a preferred embodiment of the invention, the fruit extract is present in a weight quantity of about 40%, with respect to the total weight of the composition.

Preferably, the orange extract used in the composition is obtained by microfiltration of orange juice and is intended to completely replace any aqueous content in the composition. The suggested dosages with reference to different cosmetic products are listed below:
- face or body creams: from 10% to 50% by weight of the total weight,
- lotions or toners: from 20% to 50% by weight of the total weight,
- sun creams: from 5% to 20% by weight of the total weight,
- rinsing products: from 20% to 40% by weight of the total weight,
- make-up products: from 20% to 50% by weight of the total weight,
- hair products: from 10% to 40% by weight of the total weight,
- hygiene products: from 10% to 40% by weight of the total weight.

The orange extract as per the present invention also allows giving at least one beneficial action to the epidermis. Such beneficial action is, normally, able to maintain the functional efficiency of the epidermis. In particular, the orange extract is selected to protect the skin from urban pollution thanks to its antioxidant properties that help to strengthen the skin's defenses. The orange extract also gives freshness, softness and contains vitamins and minerals that promote the renewal of the epidermis by increasing its tone and maintaining the elasticity thereof.

Moreover, the orange extract is obtained from organic crops of Mediterranean origin, certified according to the European Community REG. 889/2008 EC legislation.

In the composition according to the invention, the content of sodium lauryl ether sulfate is comprised between 20% and 60%, preferably between 35% and 45% by weight of the total weight of the composition. In an embodiment of the present invention, the content of sodium lauryl ether sulfate is present in a weight quantity of about 40%, with respect to the total weight of the composition.

Preferably, the composition contains between 2% and 20% by weight of glycerine of the total weight, preferably about 9.9%.

Preferably, the composition contains between 2% and 20% by weight of betaine of the total weight, preferably about 6%.

Preferably, the composition contains between 0.1% and 1% by weight of cocamide DEA of the total weight, preferably about 0.6%.

Preferably, the composition contains between 1% and 2% by weight of sodium chloride of the total weight, preferably about 1.8%.

Preferably, the composition contains between 0.1% and 0.9% by weight of perfumed excipient of the total weight, preferably about 0.6%.

Preferably, the composition contains between 0.5% and 1.5% by weight of benzyl alcohol or dehydroacetic acid of the total weight, preferably about 1%.

Preferably, the composition contains between 0.01% and 0.03% by weight of disodium calcium ethylenediaminetetraacetate of the total weight, preferably about 0.02%.

Preferably, the composition contains between 0.05% and 0.15% by weight of an of citric acid of the total weight, preferably about 0.08%.

Only by way of absolutely yet non-limiting example of the possible variants within the reach of the technician of the sector, the composition is illustrated below, in general terms, of a preferred mixture of ingredients to achieve the desired composition according to the invention, through appropriate mixing, according to known methods, of the components in appropriate mixing means, commonly used in the sector.

| Substance | % | INCI Name |
|---|---|---|
| Orange extract | 40.00 | Cytrus aurantium dulcis fruit extract |
| Sodium lauryl ether sulfate | 40.00 | Sodium laureth sulfate |
| Glycerin | 9.90 | Glycerin |
| Betaine CF3 | 6.00 | Cocamide propyl betaine |
| Cocamide DEA | 0.60 | Cocamide DEA |
| Sodium Chloride | 1.80 | Sodium chloride |
| Perfumed Excipient | 0.6 | Parfum |
| Benzyl alcohol, dehydroacetic acid | 1.00 | benzyl alcohol, dehydroacetic acid |
| Disodium calcium ethylenediaminetetraacetate | 0.02 | Disodium EDTA |
| Citric acid | 0.08 | Citric acid |

The Applicant has carried out several laboratory tests and analyses that have allowed determining the analytical and microbiological values of the composition of the present invention.

In a first sampling, the analytical specifications determined the values indicated in Table 1.

**Table 1**

| Analytical specifications | | |
|---|---|---|
| | Unit | Limits |
| Appearance | - | Liquid |
| Color | - | Colorless to pale yellow |
| Odor | - | Characteristic |
| pH at 25 °C | - | 3.5 ÷ 4 |
| Density at 25 °C | g/L | 0.980 ÷ 1.050 |
| Refractive index at 25 °C | - | 1.290 ÷ 1.350 |

The first sampling also determined the microbiological values indicated in Table 2.

**Table 2**

| Microbiological analysis | | |
|---|---|---|
| | Unit | Limits |
| Total bacterial count | CFU/g | < 10 |
| Coliforms | CFU/g | < 10 |
| Yeasts and moulds | CFU/g | < 10 |

| Pathogens: | | |
|---|---|---|
| - Staphylococcus aureus | CFU/g | Absent |
| - Pseudomonas aeruginosa | CFU/g | Absent |
| - Candida albicans | CFU/g | Absent |
| - Escherichia coli | CFU/g | Absent |

As it is clear from what described above, some of the substances usable for the purpose of the present invention have at the same time more than one of the indicated characteristics: they are consequently usable for more than one purpose, that is to obtain at the same time more than one beneficial effect on the skin.

## Claims

1. Water-free cosmetic composition, comprising:
- 20% to 60% of fruit extract by weight of the total weight.

2. Cosmetic composition according to the preceding claim, wherein no addition of water is made.

3. Cosmetic composition according to any one of the preceding claims, wherein said fruit extract is derived at least partly from the pressing of oranges.

4. Cosmetic composition according to any one of the preceding claims, wherein said fruit extract is entirely derived from the pressing of oranges.

5. Cosmetic composition according to any one of the preceding claims, comprising:
- 20% to 60% of sodium lauryl ether sulphate by weight of the total weight.

6. Cosmetic composition according to any one of the preceding claims, comprising:
- 2% to 20% of glycerol by weight of the total weight.

7. Cosmetic composition according to any one of the preceding claims, comprising:
- 2% to 20% of betaine by weight of the total weight.

8. Cosmetic composition according to any one of the preceding claims, comprising:
- 0.1% to 1% of cocamide DEA by weight of the total weight,
- 1% to 3% of sodium chloride by weight of the total weight,
- 0.1% to 0.9% of perfumed excipient by weight of the total weight,
- 0.5% to 1.5% of benzyl alcohol or dehydroacetic acid by weight of the total weight,
- 0.01% to 0.03% of disodium calcium ethylenediaminetetraacetate by weight of the total weight,
- 0.05% to 0.15% of citric acid by weight of the total weight.

9. Cosmetic composition according to any one of the preceding claims, comprising:
- 35% to 45% of orange extract by weight of the total weight,
- 35% to 45% of sodium lauryl ether sulphate by weight of the total weight,
- 9% to 11% of glycerine by weight of the total weight,
- 5% to 7% of betaine by weight of the total weight,
- 0.5% to 0.7% of cocamide DEA by weight of the total weight,
- 1.7% to 1.9% of sodium chloride by weight of the total weight,
- 0.5% to 0.7% of perfumed excipient by weight of the total weight,
- 0.9% to 1.1% of benzyl alcohol or dehydroacetic acid by weight of the total weight,
- 0.01% to 0.03% of disodium calcium ethylenediaminetetraacetate by weight of the total weight,
- 0.07% to 0.09% of citric acid by weight of the total weight.

10. Procedure for the preparation of a cosmetic composition in accordance with claims 1 to 6, comprising the phase of mixing the following: orange extract, sodium lauryl ether sulphate, glycerine, betaine, cocamide DEA, sodium chloride, perfumed excipient, benzyl alcohol or dehydroacetic acid, disodium calcium ethylenediaminetetraacetate and citric acid.

11. Procedure for the preparation according to the preceding claim, wherein the mixing phase is carried out at a temperature of about 25°C.

12. Use of a composition according to any one of claims 1 to 6, to carry out a cosmetic treatment of hydration of the epidermis.

13. Use of a composition according to the preceding claim, wherein said cosmetic use is made on the face, body and/or hands.
